(19) Europäisches Patentamt
European Patent Office
Office européen des brevets

(11) Publication number: 0 137 212 A2

(12) # EUROPEAN PATENT APPLICATION

(21) Application number: 84109563.1

(22) Date of filing: 10.08.84

(51) Int. Cl.[4]: A 61 K 47/00
A 61 K 9/06

(30) Priority: 12.08.83 JP 148547/83

(43) Date of publication of application:
17.04.85 Bulletin 85/16

(84) Designated Contracting States:
CH DE FR GB IT LI NL SE

(71) Applicant: OTSUKA PHARMACEUTICAL CO., LTD.
9, Kandatsukasacho 2-chome
Chiyoda-ku Tokyo 101(JP)

(72) Inventor: Akashi, Susumu
2-38, Nishinokoshi Yagura Otsu-cho
Naruto-shi Tokushima-ken(JP)

(72) Inventor: Kadowaki, Tomonori
1119-1, Kizu Muya-cho
Naruto-shi Tokushima-ken(JP)

(72) Inventor: Nishibayashi, Tohru
463-10, Kagasuno Kawauchi-cho
Tokushima-shi Tokushima-ken(JP)

(72) Inventor: Sonoda, Akio
13-4, 1-chome Higashimachi Hotarugaike
Toyonaka-shi Osaka-fu(JP)

(74) Representative: Vossius Vossius Tauchner Heunemann Rauh
Siebertstrasse 4 P.O. Box 86 07 67
D-8000 München 86(DE)

(54) **Ointment bases and ointments.**

(57) This invention provides an ointment base containing, as a solvent for the medicament, an imidazolidinone derivative represented by the formula

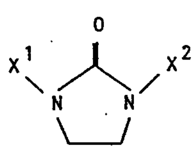

wherein $X^1$ and $X^2$ each represent a lower alkyl group, and an ointment comprising the medicament and the ointment base.

EP 0 137 212 A2

Croydon Printing Company Ltd.

# OINTMENT BASES AND OINTMENTS

## Technical Field

This invention relates to ointment bases and ointments and particularly to ointment bases and ointments for topical applications of medicaments.

## Background Art

An ointment is a semi-solid preparation intended for topical applications and comprising an ointment base and a medicament as its active component. Ointment preparations are spread over or rubbed into the skin or mucous membrane to distribute the medicament to the intended part. Therefore, an important requirement for ointments is that the active component be rapidly and reliably distributed to the affected part. It is also important that the ointment not irritate the skin or be toxic. Moreover, it is desirable that the ointment remain stable in storage over long periods.

The selection of an ointment base is one of the most important factors in satisfying these basic requirements, and the therapeutic effect of the active component itself depends on this selection. Many conventional ointments contain petrolatum, fat, glycerin or similar substances as the ointment base. However, certain medicaments, for example steroids, are only

slightly soluble in petrolatum and similar ointment bases, resulting in the undissolved portion of the medicament being merely physically dispersed in the ointment base as finely divided particles. Since these insoluble medicaments are dispersed poorly in the ointment matrix and not properly transferred to the affected part, their therapeutic effect is hindered. If a solvent is used that can dissolve these medicaments to a high concentration, the medicament can be fully dispersed or dissolved in the ointment base and more effectively transferred to the affected part to take full advantage of its therapeutic effect. Solvents conventionally used for this purpose include alcohols, propylene glycol, glycol ethers, and polyethylene glycols, but these solvents often fail to give satisfactory results.

## Disclosure of Invention

The present inventors have researched ointments and ointment bases and have found that a specific class of imidazolidinone derivatives, which have never been used for this purpose before, have outstanding properties as solvents for active components in ointment bases. These derivatives can effectively dissolve medicaments that conventional solvents cannot satisfactorily dissolve and disperse them in the ointment base to facilitate

sufficient transfer to the affected part. They can also dissolve a broad range of other medicaments to a considerable extent. The present invention is based on these novel findings.

The present invention provides an ointment base characterized by imidazolidinone derivatives that act as the solvent for the active component and are represented by the formula

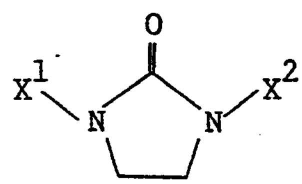

(I)

wherein $X^1$ and $X^2$ each represent a lower alkyl group.

The imidazolidinone derivatives of the formula (I) are known compounds but have not been previously used as solvents for active components in an ointment base. The present inventors are the first ones to discover that the above imidazolidinone derivatives act as ideal solvents for active components in an ointment base.

The ointment base of the present invention is basically prepared by mixing petrolatum, polysiloxane and/or similar oft-used principal ointment components with an imidazolidinone derivative of the formula (I) and, when required, other major ointment additives such as surfactants, thickeners, penetrants, adjuvants for

the principal ointment components, preservatives or antioxidants.

Conventional principal ointment components can be used in the ointment base of the present invention with few restrictions. Examples of suitable principal ointment components are petrolatum, polysiloxane, and their mixtures. Petrolatums that can be used include all paraffins ranging in viscosity from mineral oil to paraffin waxes, but the preferred petrolatum is white petrolatum. The polysiloxanes (also known as silicones) suitable for use in the present invention has a viscosity in the range of 0.5 to $10^6$ centistokes. The polysiloxanes include those with organic moieties attached such as lower alkyl, lower alkenyl, phenyl and alkyl-substituted phenyl. The amount of petrolatum, polysiloxane or other principal ointment components is decided according to the amounts in which the principal ointment components are used in conventional ointment bases and normally ranges from 30 to 99.9% by weight, preferably 60 to 99.9% by weight, more preferably 80 to 99.5% by weight, based on the weight of the ointment base.

In preparing the ointment base of the present invention, it is imperative that an imidazolidinone derivative of the formula (I) be used as the solvent. Representative of the lower alkyl

groups indicated by $X^1$ and $X^2$ in the formula (I) are methyl, ethyl, propyl, isopropyl, butyl, tert-butyl, isobutyl, and sec-butyl. The amount of the imidazolidinone derivative used is decided according to the amounts in which commonly used solvents are used in conventional ointment bases and normally ranges from 0.1 to 30% by weight, preferably 0.5 to 30% by weight, more preferably 0.5 to 10% by weight, based on the weight of the ointment base.

An imidazolidinone derivative of the formula (I) according to the present invention can be used singly or, when required, in conjunction with, as a cosolvent, one or more of the commonly used solvents. When combined with a cosolvent, there is no restriction on the amount of the imidazolidinone derivative used, but 30% or more by weight is preferred, based on the total weight of the imidazolidinone derivative and the cosolvent. There is no restriction on the combined amount of the derivative and the cosolvent, and it can be decided according to the total amounts in which the solvents and cosolvents are used in conventional ointment bases. The amount normally ranges from 0.1 to 30% by weight, preferably 0.5 to 30% by weight, more preferably 0.5 to 10% by weight, based on the weight of the ointment base. Examples of useful cosolvents are monohydric

alcohols having 1 to 22 carbon atoms such as methanol, ethanol, propanol, cetyl alcohol, stearyl alcohol, 2-phenylethyl alcohol, benzyl alcohol, and the like; dihydric or polyhydric alcohols such as propylene glycol, glycerin, and the like; lower alkyl ethers of ethylene or diethylene or propylene or dipropylene glycol, etc.; polyethylene glycols or polypropylene glycols having molecular weights of 100 to 20,000; esters of aliphatic monobasic or dibasic acids having 2 to 22 carbon atoms and monohydric alcohols having 1 to 20 carbon atoms such as isopropyl myristate, myristyl myristate, cetyl stearate, methyl stearate, isopropyl sebacate, and the like; sterols such as cholesterol, lanolin, and the like.

When required, the ointment base of the present invention can contain one or more kinds of surfactants to facilitate the dispersion of the solvent or cosolvent in the ointment base and to stabilize the same or to improve the spreadability of the ointment base. Suitable surfactants can be any of those used in conventional ointment bases and include anionic, cationic, amphoteric and nonionic surfactants that are pharmaceutically acceptable for topical applications. Typical examples of suitable surfactants for the ointment bases of the present invention are given below.

Exemplary of useful anionic surfactants are

salts of sulfonates such as sodium dodecylbenzenesulfonate, sodium lauryl sulfate, etc.

Representative of useful cationic surfactants are quaternary ammonium salts such as cetyl trimethyl ammonium bromide, benzalkoniumchloride, etc.

Illustrative of useful nonionic surfactants are ethers such as condensation products of alkylphenols with 6 to 20 moles of ethylene oxide, the phenols being monoalkylated, dialkylated or polyalkylated with alkyl side chains having 5 to 18 carbon atoms and the corresponding naphthalene or diphenyl compounds, polyoxyethylene and polyoxyethylene-polyoxypropylene copolymers, esters such as compounds which can be represented by the formula $RCOOR^1$ (wherein R is a long hydrocarbon chain derived from a fatty acid having from 12 to 22 carbon atoms and $R^1$ is a polyhydric alcohol), e.g. glyceryl monostearate, diethylene glycol monolaurate, sorbitan fatty acid esters derived, for example, from lauric, palmitic, stearic and oleic acids, ether-esters wherein polyoxyethylene chains are found with an unreacted hydroxy group of esters of fatty acids and polyhydric alcohols, fatty acid amides such as lauroyl diethanolamide, etc.

Exemplary of useful amphoteric surfactants are those having amino and carboxy groups, e.g. dodecyl β-alanine, imidazoline derivatives such as Miranols,

those containing amino and sulfuric acid or sulfonic groups formed by condensing an alkane-sulfonamide with formaldehyde and methyltaurine, etc.

More specifically, the surfactants that can be used include sorbitan trioleate, sorbitan tristearate, sorbitan sesquioleate, glycerol monostearate, sorbitan monostearate, sorbitan monopalmitate, sorbitan monolaurate, polyoxyethylene lauryl ether, polyethylene glycol 400 monostearate, triethanolamine oleate, polyethylene glycol 400 monolaurate, polyoxyethylene sorbitan monostearate, polyoxyethylene sorbitan monooleate, polyoxyethylene sorbitan monolaurate, sodium oleate, potassium oleate, sodium lauryl sulfate, lauroyl imidazoline, sodium dodecylbenzene sulfate, sodium monoglyceride sulfate, sodium oleyl taurate, sodium dioctyl sulfosuccinate, lauryl polyglycol ether; sodium dibutylnaphthalenesulfonate, alkyl phenol polyglycol ether, sorbitan monolaurate polyglycol ether, sulfonated castor oil, tall oil polyglycol ester, alkyl dimethyl benzylammonium chloride, alkyl naphthalene pyridinium chloride, cetyl dimethyl ethylammonium bromide, alkyl dimethyl chlorobenzylammonium chloride, dibutyl phenyl phenol sulfonate, sulfonated methyl oleylamide, sorbitan monolaurate polyglycol ether, polyglycol oleate, sodium lauryl sulfoacetate, sodium 2-ethylhexanol sulfate,

sodium 7-ethyl-2-methylundecanol-4 sulfate, sodium 3,9-diethyltridecanol-6 sulfate, sodium lauryl sulfonate and N-(sodium sulfoèthyl)oleamide, etc. The amount of the surfactant used is suitably decided according to the amount in which the surfactants are used in conventional ointment bases and is normally 45% or less by weight, preferably 0.1 to 10% by weight, more preferably 0.1 to 5% by weight, based on the weight of the ointment base.

A thickener, when required, can be incorporated in the ointment base of the present invention to adjust its viscosity and consistency. Suitable thickeners include colloidal alumina, colloidal silica, alginic acid and derivatives thereof, Carbopol (carboxyvinyl polymer), cellulose derivatives such as Klucel (cellulose ethers), Methocel (methyl cellulose), Natrosol (hydroxyethyl cellulose), sodium carboxymethyl cellulose, gelatin, gums such as agar, tragacanth, acacia gum, guar gum, and the like, and resins such as ethyleneoxide polymers. The amount of the thickener used is decided in accordance with the kinds and amounts of the principal ointment components, solvents, and the like, and is usually 20% or less by weight of the ointment base.

Adjuvants for the principal ointment components can be incorporated into the ointment base of the present invention, when required, to increase the spreadability

of the ointment base and to enhance the feel of the ointment on the skin. Examples of useful such adjuvants are beeswax, spermaceti, fatty acid having 10 to 22 carbon atoms, etc.

The ointment base of the present invention can also include, when required, a penetrant to enhance the absorption of the active component. Examples of useful penetrants are dialkyl sulfoxide having up to 22 carbon atoms in each alkyl group, e.g., dimethylsulfoxide, dimethylformamide, dimethylacetamide, tetrahydrofuran, and tetrahydrofurfuryl alcohol. The ointment base of the invention can also contain penetration aids such as hydrocarbons including squalene and squalane, acetylated lanolin fractions, etc.

The ointment base of this invention can also contain suitable preservatives or antioxidants such as methyl, ethyl, propyl, and butyl esters of parahydroxybenzoic acid, propyl gallate, sorbic acid and its sodium and potassium salts, propionic acid and its calcium and sodium salts, 6-acetoxy-2,4-dimethyl-m-dioxane, 2-bromo-2-nitropropane-1,3-diol, and salicylanilides such as dibromosalicylanilide, tribromosalicylamilides, Cinaryl 100 and 200 or Dowicil 100 and 200 (Cis isomer of 1-(3-chloroallyl-3,5,7-triaza-1-azaniadamantane chloride), hexachlorophene, sodium benzoate, citric acid, ethylene

diaminetetraacetic acid and its alkali metal and alkaline earth metal salts, butyl hydroxyanisol, butyl hydroxytoluene, phenolic compounds such as chloro- and bromocresols, quaternary ammonium compounds such as benzalkonium chloride, chlorobutanol, iodochlorohydroxyquinolin, tocopherol, etc. The amount of the preservative and antioxidant used is 0.1 to 2% by weight of the ointment base.

The ointment base of the present invention can be generally prepared by the conventional method described below. For example, the components as stated above are thoroughly mixed together at ambient or higher temperatures. It is preferable that the imidazolidinone derivative of the formula (I) or a mixture of the derivative of the formula (I) with a cosolvent is thoroughly mixed with a surfactant and/or a thickener for dissolution while each of the components are in a liquid state. In this case, if required for dissolution, the components can be heated to, for example, 60 to 85°C while stirring. Subsequently petrolatum or like principal ointment component and the other components are heated until these components become a liquid (e.g., 60 to 85°C). Then the solvent mixture is gradually added to the liquid components and the resulting mixture is cooled with good

agitation to obtain the present ointment base. If the ointment base thus obtained has a low viscosity or separation of phase occurs in the ointment base, these problems can be solved, for example, by adding the thickener or surfactant to the ointment base and heating the mixture with stirring or increasing the amount of the thickener or surfactant used in the foregoing process.

The present ointment base can be used for topical applications of various medicaments by virtue of the use of the imidazolidinone derivative of the formula (I) as the solvent. The medicaments which can be admixed with the present ointment base are steroidal or non-steroidal anti-inflammatory agents, therapeutic agents for eye diseases, cardiotonics, virucides, antifungals, antiallergic agents, hormones, analgesics, etc.

Ointments containing the foregoing medicaments can be formulated by the conventional methods. For example, the foregoing components are thoroughly mixed together at ambient or higher temperatures. It is preferable to add the medicaments to the imidazolidinone derivative of the formula (I) or a solvent mixture of the derivative (I) with a cosolvent or a combined mixture of of the above solvent or solvent mixture with a surfactant and/or thickener, and then mix these together while in the liquid state. In this case, if required for dissolution, the components

can be heated to, for example, 60 to 85°C, while stirring. Subsequently petrolatum or like principal ointment component and the other components are heated until they become liquid (e.g., 60 to 85°C). Next, the mixture containing the medicament is gradually added to the liquid components and the resulting admixture is cooled with sufficient stirring to prepare an ointment. Alternatively an ointment can be formulated by adding the medicament to the ointment base of the present invention with heating, and then thoroughly stirring the mixture while cooling. Further the mixture can be mechanically agitated at the intermediate or final stage of the process to make the mixture more uniform. The mechanical agitation can be conducted by use of a conventional devices such as a propeller mixer, colloid mill, homogenizer, roller mill, sonic mixer, etc.

The ointment base of the present invention is particularly suitable for use in the formulation of an ointment containing a steroid. Examples of such steroids are:

anti-inflammatory steroids of the formula (A) as given below which are disclosed in Japanese Unexamined Patent Publication No.45200/1982;

9-fluoro-11β,17,21-trihydroxy-16α-methylpregna-1,4-diene-3,20-dione;

11β,17,21-trihydroxypregn-4-ene-3,20-dione;

9-fluoro-11β,17,21-trihydroxy-16β-methylpregna-1,4-diene-3,20-dione (betamethasone);

11β,21-dihydroxypregn-4-ene-3,10-dione-17-butyrate;

6α,9α-difluoro-11β,16α,17α,21-tetrahydroxypregna-1,4-diene-3,10-dione-16α,17α-acetonide, etc.

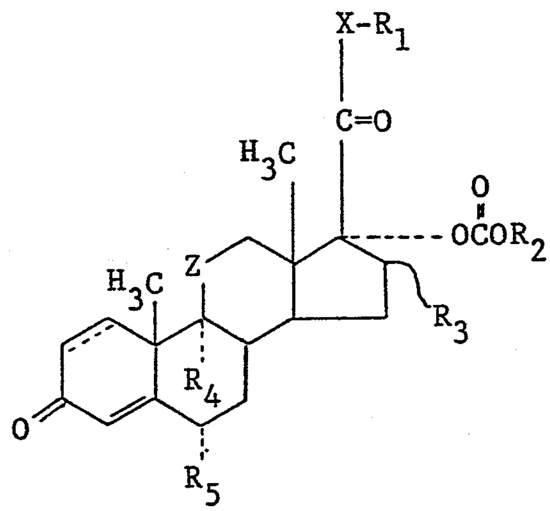

(A)

wherein

$R_1$ is $C_1$-$C_{10}$ alkyl; $C_2$-$C_{10}$ (monohydroxy or polyhydroxy)alkyl; $C_1$-$C_{10}$ (monohalo or polyhalo)alkyl; or $-CH_2COOR_6$ wherein $R_6$ is unsubstituted or substituted $C_1$-$C_{10}$ alkyl, $C_3$-$C_8$ cycloalkyl, $C_3$-$C_8$ cycloalkenyl or $C_2$-$C_{10}$ alkenyl, the substituents being selected from the group consisting of halo, lower alkoxy, lower alkylthio, lower alkylsulfinyl, lower alkylsulfonyl, $-NH\overset{O}{\overset{\|}{C}}-(C_1\text{-}C_{10}\text{ alkyl})$ and $-O\overset{O}{\overset{\|}{C}}-(C_1\text{-}C_{10}\text{ alkyl})$, or $R_6$ is unsubstituted or substituted phenyl or benzyl, the

substituents being selected from the group consisting of lower alkyl, lower alkoxy, halo, carbamoyl, lower alkoxycarbonyl, lower alkanoyloxy, lower haloalkyl, mono(lower alkyl)amino, di(lower alkyl)amino, mono(lower alkyl)carbamoyl, di(lower alkyl)carbamoyl, lower alkylthio, lower alkylsulfinyl and lower alkylsulfonyl; or $R_1$ is $-CH_2CONR_7R_8$ wherein $R_7$ and $R_8$, which can be the same or different, are each hydrogen, lower alkyl, $C_3$-$C_8$ cycloalkyl, phenyl or benzyl, or $R_7$ and $R_8$ are combined such that $-NR_7R_8$ represents the residue of a saturated monocyclic secondary amine; or $R_1$ is unsubstituted or substituted phenyl or benzyl, the substitutents being selected from the group of phenyl and benzyl substituents defined hereinabove with respect to $R_6$; or $R_1$ is $-\underset{R_9}{\underset{|}{CH}}-Y-$(lower alkyl) wherein Y is -S-, -SO-, $-SO_2-$ or -O- and $R_9$ is hydrogen, lower alkyl or phenyl, or $R_9$ and the lower alkyl group adjacent to Y are combined so that $R_1$ is a cyclic system of the type

-CH——Y
 |alkylene|

wherein Y is defined as above and the alkylene group contains 3 to 10 carbon atoms, of which at least 3 and no more than 6 are ring atoms; or $R_1$ is $-\underset{R_{10}}{\underset{|}{CH}}-O\overset{O}{\overset{\|}{C}}R_6$ wherein $R_6$ is defined as

hereinabove and $R_{10}$ is hydrogen, lower alkyl, phenyl or haloalkyl;

$R_2$ is unsubstituted or substituted $C_1$-$C_{10}$ alkyl, $C_3$-$C_8$ cycloalkyl, $C_3$-$C_8$ cycloalkenyl or $C_2$-$C_{10}$ alkenyl, the substituents being selected from the group consisting of halo, lower alkoxy, lower alkylthio, lower alkylsulfinyl, lower alkylsulfonyl, $-NH\overset{\overset{O}{\|}}{C}-(C_1-C_{10}\text{ alkyl})$ and $-O\overset{\overset{O}{\|}}{C}-(C_1-C_{10}\text{ alkyl})$, or $R_2$ is unsubstituted or substituted phenyl or benzyl, the substituents being selected from the group consisting of lower alkyl, lower alkoxy, halo, carbamoyl, lower alkoxycarbonyl, lower alkanoyloxy, lower haloalkyl, mono(lower alkyl)amino, di(lower alkyl)-amino, mono(lower alkyl)carbamoyl, di(lower alkyl)-carbamoyl, lower alkylthio, lower alkylsulfinyl and lower alkylsulfonyl;

$R_3$ is hydrogen, α-hydroxy, β-hydroxy, α-methyl, β-methyl, $=CH_2$, or α- or β $-O\overset{\overset{O}{\|}}{C}OR_2$ wherein $R_2$ is as defined hereinabove;

$R_4$ is hydrogen, fluoro or chloro;

$R_5$ is hydrogen, fluoro, chloro or methyl;

X is -O- or -S-;

Z is carbonyl or β-hydroxymethylene;

and the dotted line in ring A indicates that the 1,2 linkage is saturated or unsaturated.

When at least one of $R_1$ and $R_2$ is halo-alkyl, a quaternary ammonium salt of the compound of the formula (A) is also included.

The amount of the medicament admixed with the ointment base of the present invention varies depending on the type of the medicament, its efficacy and its intended use but is generally decided based on the therapeutically effective amount. The amount of the medicament usually ranges from 0.01 to 5.0 parts by weight, and in the case of steroids, preferably from 0.01 to 1.0 part by weight, more preferably 0.05 to 0.2 part by weight, per 100 parts by weight of the ointment base.

Given below are Examples, Comparison Examples and Test Examples in which the term "Steroid A" refers to chloromethyl 17α-ethoxycarbonyloxy-11β-hydroxyandrosta-1,4-diene-3-one-17β-carboxylate and the term "Steroid B" refers to chloromethyl 17α-ethoxycarbonyloxy-9α-fluoro-11β-hydroxy-16α-methylandrosta-1,4-diene-3-one-17β-carboxylate.

Example 1

(1) An ointment base with the following composition was prepared.

| Components | Amount (g) |
|---|---|
| 1,3-Dimethyl-2-imidazolidinone | 3.0 |
| Propylene glycol | 3.0 |
| Isopropyl myristate | 2.0 |
| White petrolatum | 92.0 |

A mixture of the 1,3-dimethyl-2-imidazolidinone and propylene glycol was heated to 75°C. Then the white petrolatum and isopropyl myristate were heated to 70°C and mixed. The two mixtures were combined with stirring, and the resulting admixture was cooled with further agitation to room temperature to obtain the ointment base of the present invention.

(2) An ointment was produced by repeating the same procedure as in (1) above except that 1.0 g of Steroid B was dissolved in the mixture of the 1,3-dimethyl-2-imidazolidinone and propylene glycol prior to heating.

Example 2

(1) An ointment base with the following composition was prepared.

| Components | Amount (g) |
|---|---|
| 1,3-Dimethyl-2-imidazolidinone | 5.0 |
| Polyoxyethylene sorbitan monostearate (20 M) (trademark "NYKKOL TS-10", product of Nikko Chemicals Kabushiki Kaisha, Japan) | 2.0 |
| Sorbitan sesquioleate | 1.0 |

| | |
|---|---|
| Polyethylene glycol 400 | 2.0 |
| Stearyl alcohol | 2.0 |
| White petrolatum | 83.0 |
| Lanolin | 5.0 |

The 1,3-dimethyl-2-imidazolidinone, polyethylene glycol 400 and polyoxyethylene sorbitan monostearate (20 M) (1.0 g) were mixed while heating at 70°C. Separately, the sorbitan sesquioleate, polyoxyethylene sorbitan monostearate (20 M) (1.0 g), stearyl alcohol, lanolin and white petrolatum were mixed while heating at 70°C. The two mixtures were mixed while heating at 70°C until the resulting admixture became uniform. Thereafter further agitation continued to cool the admixture to room temperature, giving the ointment base of the present invention.

(2) An ointment was prepared in the same manner as in (1) above except that in place of the mixture of the 1,3-dimethyl-2-imidazolidinone, polyethylene glycol 400 and polyoxyethylene sorbitan monostearate (20 M), a solution of 0.1 g Steroid B in the mixture was used.

Example 3

(1) An ointment base with the following composition was prepared.

| Components | Amount (g) |
|---|---|
| 1,3-Dimethyl-2-imidazolidinone | 10.0 |
| Sorbitan monolaurate | 3.0 |
| Polyoxyethylene sorbitan monooleate (20 M) (trademark "NYKKOL TO-10", product of Nikko Chemicals Kabushiki Kaisha, Japan) | 5.0 |
| Solid paraffin (m.p. 56 to 58°C) | 5.0 |
| Liquid paraffin (viscosity 350 cs) | 20.0 |
| Beeswax | 5.0 |
| White petrolatum | 52.0 |

The 1,3-dimethyl-2-imidazolidinone and polyoxyethylene sorbitan monooleate were mixed and stirred while heating at 70°C. The solid paraffin, liquid paraffin, beeswax and white petrolatum were fully mixed together while heating at 70°C. The former mixture was added to the latter mixture at the same temperature as above while stirring and the resulting admixture was stirred until it became uniform. The admixture was then agitated until it cooled to room temperature, giving the ointment base of the present invention.

(2) An ointment was prepared in the same manner as in (1) above except that in place of the mixture of the 1,3-dimethyl-2-imidazolidinone and polyoxyethylene sorbitan monooleate, a solution of 0.1 g of Steroid B in the mixture was used.

Example 4

(1) An ointment base with the following composition was prepared.

| Components | Amount (g) |
|---|---|
| 1,3-Dimethyl-2-imidazolidinone | 11.0 |
| Liquid paraffin (viscosity 350 cs) | 45.0 |
| Beeswax | 5.0 |
| Isopropyl myristate | 24.0 |
| Colloidal silica (trademark "Aerozil 300", product of Nihon Aerozil Kabushiki Kaisha, Japan) | 15.0 |

The 1,3-dimethyl-2-imidazolidinone was heated to 70°C and added to a hot mixture (70°C) of the liquid paraffin, beeswax and isopropyl myristate with stirring. The colloidal silica was gradually added to the mixture with stirring and the resulting admixture was stirred until it became uniform. The resulting admixture was agitated until it cooled to room temperature, giving the ointment base of the present invention.

(2) An ointment was formulated in the same manner as in (1) above except that a solution of 0.1 g of Steroid B in the 1,3-dimethyl-2-imidazolidinone was used in place of the 1,3-dimethyl-2-imidazolidinone.

Example 5

(1) An ointment base with the following composition was prepared.

| Components | Amount (g) |
|---|---|
| 1,3-Dimethyl-2-imidazolidinone | 4.0 |
| Polysiloxane (viscosity 1000 cs) | 30.0 |
| Propylene glycol | 2.0 |
| Sorbitan sesquioleate | 3.0 |
| White petrolatum | 61.0 |

The white petrolatum and polysiloxane were heated together to a temperature of 65 to 70°C and thoroughly mixed with stirring. The sorbitan sesquioleate was added to the mixture and mixed therewith. Thereto was added a uniform mixture of the 1,3-dimethyl-2-imidazolidinone and propylene glycol with stirred and the resulting mixture was agitated until it became uniform and cooled to room temperature, whereby the ointment base of the present invention was obtained.

(2) An ointment was produced in the same manner as in (1) above except that a solution of 0.1 g of Steroid B in the 1,3-dimethyl-2-imidazolidinone was used in place of the 1,3-dimethyl-2-imidazolidinone.

Example 6

An ointment was prepared in the same manner as in Example 2 (2) except that 0.01 g of Steroid B was used.

Example 7

Six kinds of ointments were prepared in the same manner as in Examples 1 to 6 except that Steroid A was used in place of Steroid B.

Example 8

(1) An ointment base with the following composition was prepared.

| Components | Amount (g) |
|---|---|
| White petrolatum | 95.0 |
| 1,3-Dimethyl-2-imidazolidinone | 5.0 |

The 1,3-dimethyl-2-imidazolidinone was heated to 70°C and added to the white petrolatum heated to 70°C, while stirring and the resulting mixture was stirred until it became uniform. The admixture was agitated until it cooled to room temperature, affording the ointment base of the present invention, which is hereinafter referred to as "Ointment Base-I".

(2) An ointment was produced in the same manner as in (1) above except that a solution of 0.1 g of Steroid A in the 1,3-dimethyl-2-imidazolidinone was used in place of the 1,3-dimethyl-2-imidazolidinone. The resulting ointment is hereinafter referred to as "Ointment A".

Example 9

An ointment was prepared in the same manner as in Example 8 (2) except that Steroid B was used in

place of Steroid A. The ointment is hereinafter referred to as "Ointment B".

Example 10

(1) An ointment base of the following composition was prepared.

| Components | Amount (g) |
|---|---|
| White petrolatum | 98.0 |
| 1,3-Dimethyl-2-imidazolidinone | 2.0 |

The 1,3-dimethyl-2-imidazolidinone was heated to 70°C and added to the white petrolatum heated to 70°C while stirring and the resulting mixture was stirred until it became uniform. The mixture was then agitated until it cooled to room temperature, affording the ointment base of the present invention.

(2) An ointment was formulated in the same manner as in (1) above except that in place of the 1,3-dimethyl-2-imidazolidinone, a solution of 0.1 g of Steroid A in the 1,3-dimethyl-2-imidazolidinone was used. The ointment is hereinafter referred to as "Ointment C".

Example 11

An ointment was formulated in the same manner as in Example 10 (2) except that Steroid B was used in place of Steroid A. The ointment was hereinafter referred to as "Ointment D".

Example 12

An ointment was formulated in the same manner as in Example 10 (2) except that 0.12 g of betamethasone was used in place of Steroid A. The ointment is hereinafter referred to as "Ointment E".

Comparison Example 1

A 100 g quantity of white petrolatum was heated to 70°C and 0.12 g of betamathasone was added thereto with stirring and the mixture was stirred until it became uniform. The mixture was then agitated until it cooled to room temperature, giving an ointment which is hereinafter referred to as "Ointment F".

Example 13

Ointments were produced in the same manner as in Examples 1 to 5 except that the followng medicaments were used.

° chloromethyl 17α-ethoxycarbonyloxy-9α-fluoro-11β-hydroxy-16β-methylandrosta-1,4-diene-3-one-17β-carboxylate;

° chloromethyl 9α-fluoro-11β-hydroxy-17α-isopropoxycarbonyloxy-16α-methylandrosta-1,4-diene-3-one-17β-carboxylate;

° chloromethyl 17α-cyclohexyloxycarbonyloxy-11β-hydroxyandrost-4-ene-3-one-17β-carboxylate;

° methyl 11β-hydroxy-17α-isopropoxycarbonyloxyandrost-4-ene-3-one-17β-carboxylate;

° ethoxymethyl 11β-hydroxy-17α-isopropoxycarbonyloxy-androst-4-ene-3-one-17β-carboxylate;

° chloromethyl 17α-benzyloxycarbonyloxy-11β-hydroxy-androst-4-ene-3-one-17β-carboxylate;

° 1-chloroethyl 11β-hydroxy-17α-isopropoxycarbonyloxy-androst-4-ene-3-one-17β-carboxylate;

° ethoxycarbonylmethyl 11β-hydroxy-17α-isopropoxy-carbonyloxyandrost-4-ene-3-one-17β-carboxylate;

° chloromethyl 9α-fluoro-17α-isopropoxycarbonyloxy-16α-methylandrosta-1,4-diene-3,11-dione-17β-carboxylate;

° chloromethyl 9α-fluoro-11β-hydroxy-17α-methoxy-carbonyloxy-16α-methylandrosta-1,4-diene-3-one-17β-carboxylate;

° chloromethyl 9α-fluoro-11β-hydroxy-16α-methyl-17α-pentyloxycarbonyloxyandrosta-1,4-diene-3-one-17β-carboxylate;

° chloromethyl 16α,17α-di(ethoxycarbonyloxy)-6α-fluoro-11β-hydroxyandrosta-1,4-diene-3-one-17β-carboxylate;

° fluoromethyl 17α-ethoxycarbonyloxy-9α-fluoro-11β-hydroxy-16α-methylandrosta-1,4-diene-3-one-17β-carboxylate;

° acetoxymethyl 17α-ethoxycarbonyloxy-11β-hydroxyandrost-4-ene-3-one-17β-carboxylate;

° 2-chloroethyl 17α-ethoxycarbonyloxy-9α-fluoro-11β-hydroxy-16α-methylandrosta-1,4-diene-3-one-17β-carboxylate;

° 8-(1-piperazinyl)-9-fluoro-5-methyl-6,7-dihydro-1-oxo-1H,5H-benzo[i,j]quinolizine-2-carboxylic acid;

° D(-)-threo-2,2-dichloro-N-[β-hydroxy-α-(hydroxymethyl)-p-nitrophenethyl]acetamide;

° 2-diphenylmethoxy-N,N-dimethylethylamine;

° 1-(o-chloro-α,α-diphenylbenzyl)imidazole;

° 4-(dimethylamino)-1,4,4a,5,5a,6,11,12a-octahydro-3,6,10,12,12a-pentahydroxy-6-methyl-1,11-dioxo-2-naphthacenecarboxyamide;

° 1,4-dimethyl-7-isopropylazulene;

° 1-(p-chlorobenzoyl)-5-methoxy-2-methyl-3-indolyl-acetic acid;

° 6-[D(-)-α-aminophenylacetamido]penicillanic acid trihydrate.

Ointments prepared by using the solvent and ointment base of the present invention were tested for properties with the results as shown below.

Steroid Solubility Test

This test was conducted to determine the maximum weight of Steroids A and B that could completely dissolve in 1 ml of various solvents at a temperature of 20°C. Table 1 below shows the results.

Table 1

| Solvent | Steroid A (mg/ml) | Steroid B (mg/ml) |
| --- | --- | --- |
| Benzyl alcohol | >25.0 | 0.92 |
| Diethyl sebacate | 6.85 | 0.28 |
| Polyethylene glycol 400 | 13.32 | 0.45 |
| 1,3-Butanediol | 1.95 | 0.05 |
| Propylene glycol | 2.7 | 0.10 |
| 1,3-Dimethyl-2-imidazolidinone | 357.4 | 52.1 |

Table 1 reveals that the 1,3-dimethyl-2-imidazolidinone exhibits an outstanding dissolving ability.

Human vasoconstrictor assay

The test was conducted with six healthy male adults. About 60 mg of each of Ointments B to F prepared above was applied to a piece of commercially available adhesive plaster for patch tests (product of Torii Yakuhin Kabushiki Kaisha, Japan). Then the pieces of adhesive plaster coated with the ointments were placed on the upper back of the male adults, and left there for 4 hours. The pieces of adhesive plaster with the ointments were all removed. Twice, i.e., at 2 and 4 hours after removal of the ointments, 4 persons evaluated the degree of blanching on the skin resulting from the

vasoconstriction effect of the medicament according to the following criteria (The Nishinihon Journal of Dermatology, Vol. 38, No.2, pp. 286 to 293, 1976):

+ : the skin shows an obvious blanching

± : the skin shows a faint blanching

- : the skin shows no discernible blanching

Thus 24 evaluations (6 adults x 4 evaluators = 24) were obtained with respect to each of the ointments. The number ($N_1$) of evaluations in which a faint blanching or an obvious blanching was observed and the number ($N_2$) of evaluations in which only an obvious blanching was observed were determined and expressed as a percentage of the total number of evaluations (100 $N_1$/24 or 100 $N_2$/24). Table 2 below shows the results.

Table 2

| | Ointment | $N_1$ (%) | $N_2$ (%) |
|---|---|---|---|
| Two hours after removal of ointment | Ointment C | 23 (95.83) | 23 (95.83) |
| | " B | 24 ( 100 ) | 21 (87.50) |
| | " D | 24 ( 100 ) | 19 (79.17) |
| | " E | 24 ( 100 ) | 24 ( 100 ) |
| | " F | 14 (58.3 ) | 9 (37.5 ) |
| Four hours after removal of ointment | Ointment C | 24 ( 100 ) | 23 (95.83) |
| | " B | 24 ( 100 ) | 23 (95.83) |
| | " D | 24 ( 100 ) | 22 (91.67) |
| | " E | 24 ( 100 ) | 24 ( 100 ) |
| | " F | 21 (87.5 ) | 16 (66.67) |

Table 2 reveals that the use of 1,3-dimethyl-2-imidazolidinone (Ointments B to E) causes significant vasoconstriction when compared with the ointment formulated without use of the solvent (Ointment F). The vasoconstrictor assay as described above is often used to check steroid-containing ointments for efficacy and the vasoconstriction in humans is known to accurately reflect clinical effect. Table 2 indicates, therefore, that the therapeutic effect of each of the medicaments is improved when 1,3-dimethyl-2-imidazolidinone is used as the solvent.

Inhibition of carrageenin-induced paw edema in rats

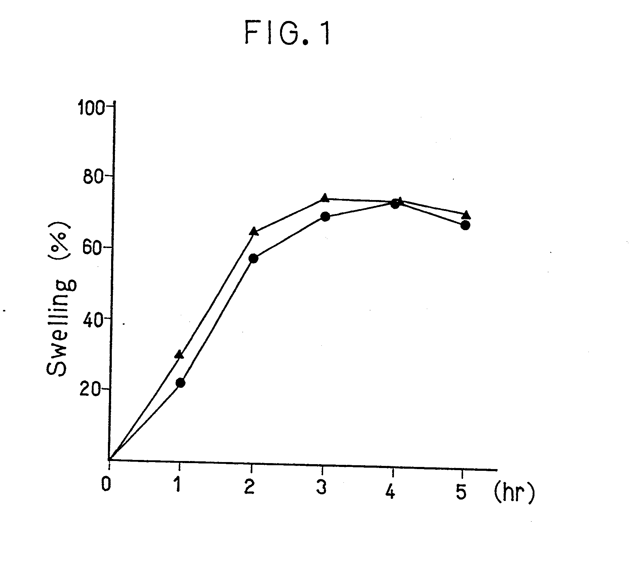

After the ointment was applied to a rat's hind paw, carrageenin serving as a phlogogen was injected into the same hind paw, and the swelling caused by the phlogogen was measured. More specifically stated, the test was carried out as given below, using groups of 5 rats (weighing 175 g). First, the right hind paw of the rat was immersed in water, and the displacement was measured thereby giving the volume of hind paw. One and 2 hours before the injection of the phlogogen (carrageenin), 200 mg of the ointment was elaborately rubbed into the right hind paw of the rat, which was then wrapped in Saran Wrap (trademark) and covered with an elastic strip of adhesive plaster. Thereafter, 0.1 ml

of a 1% solution of carrageenin was injected into the right hind paw, and the volume of the hind paw was hourly measured to determine the swelling (%) according to the following equation based on the volume thereof before the injection with the results shown in Fig. 1.

$$\text{Swelling (\%)} = \frac{V_1 - V_0}{V_0} \times 100$$

wherein $V_0$ is the volume of the right hind paw before the injection of carrageenin and $V_1$ is the volume thereof after the injection of carrageenin.

In Fig. 1, the line ●-● shows the results achieved by the application of Ointment A and the line ▲-▲ shows those attained by the application of Ointment B.

Fig. 1 reveals that Steroids A and B exhibit an anti-inflammatory activity.

Inhibition of histamine-induced vascular permeability

This test was conducted according to the method disclosed in Microbiol. Immunol. Vol. 22 (2), pp. 89 to 101, 1978 by determining the degree to which the test ointment was able to suppress the vascular permeability increased by the injection of histamine. More specifically stated, the back of rats (weighing about 200 g), 8 rats in each group, was shaved with electric clippers. A 100 mg quantity of the test

ointment was applied to each piece of adhesive plaster for patch tests, and the pieces of adhesive plaster coated with the ointment were affixed on the rat's back on one side of the mid-dorsal line and held there with and adhesive bandage. The ointment application was repeated twice at 5 hours and 3 hours prior to the histamine injection. On removal of the ointment, a physiological saline solution of histamine (10 μg/0.1 ml) was intradermally injected under ether anesthesia in an amount of 0.1 ml at each of two locations, i.e., where the ointment-coated adhesive plaster had been affixed and a location on the opposite side of the mid-dorsal line (untreated portion), immediately followed by an intravenous injection of 1% physiological saline solution of Evans Blue (dye) in an amount of 0.5 ml/body. Thirty minutes after the injections, the animals were sacrificed by exsanguination. Then the discolored skin portions were removed, each with the same size, from the back and separately immersed in 1 ml of 1 N solution of potassium hydroxide and subsequently left to stand at 37°C overnight. Thereto was added 9 ml of a mixture of 0.6 N solution of $H_3PO_4$ and acetone (5 : 13), and the resulting mixture was shaken and centrifuged (3000 rpm for 15 minutes). The absorbancy in 620 nm of the supernatant thus obtained was measured photometrically to determine the amount of the dye extravasated from each of the skin portions.

The ointment was evaluated in terms of the

inhibition ratio calculated by the following equation on the basis of the amount of the extravasated dye from the untreated portion ($W_0$) and the amount of the extravasated dye from the portion to which the ointment was applied ($W_1$):

$$\text{Inhibition ratio (\%)} = \left(1 - \frac{W_1}{W_0}\right) \times 100$$

The results are shown in Table 3 below which demonstrates that the use of 1,3-dimethyl-2-imidazolidinone as the solvent (Ointments A and B) attains a high inhibition of the histamine-induced vascular permeability, thereby producing an outstanding anti-inflammatory activity.

Table 3

| Test Ointment | Inhibition Ratio (%) |
|---|---|
| Ointment A | 40 |
| Ointment B | 49 |

Test for primary dermal irritations in rabbits

The back of 9 rabbits was shaved with electric clippers. The next day 100 mg of the test ointment or petrolatum was applied to each of the exposed skin areas of the back in a circular layer about 2 cm in diameter thereon. The preparations were applied to different locations on each rabbit in consideration of their individual differences and the differences in the sensitivity of the exposed skin area depending on its

location. After the application, the skin areas coated with the preparation were observed at 3, 6, 24 and 48 hours to evaluate the degree to which erythema, eschar and edma had formed based on the scale for scoring dermal irritation according to Draize at al. (Yakuri to Chiryo (Japanese Pharmacology and Therapeutics), Vol. 9, No.8, Aug., pp. 95 to 105, 1981). The score totals are the index of intensity of irritation and are shown in Table 4 below. It is seen from Table 4 that Ointment B induces little or no dermal irritation.

The scale according to Draize et al is as follows.

1) Erythema and eschar formation

| | Score |
|---|---|
| No erythema | 0 |
| Very slight erythema | 1 |
| Well defined erythema | 2 |
| Slightly severe erythema | 3 |
| Servere erythema | 4 |

2) Edema formation

| | Score |
|---|---|
| No edema | 0 |
| Very slight edema | 1 |
| Well defined edema | 2 |
| Slightly severe edema | 3 |
| Severe edema | 4 |

The scores were calculated by adding the scores for erythema and eschar formation in 1) and those for edema formation in 2).

Table 4

| | Time (hr) | Score 0 | 1 | 2 | 3 | Number of Positive | Mean of Score |
|---|---|---|---|---|---|---|---|
| Ointment B | 3 | 6 | 3 | 0 | 0 | 3 | 0.3 |
| | 6 | 7 | 2 | 0 | 0 | 2 | 0.2 |
| | 24 | 9 | 0 | 0 | 0 | 0 | 0 |
| | 48 | 9 | 0 | 0 | 0 | 0 | 0 |
| Petrolatum | 3 | 7 | 2 | 0 | 0 | 2 | 0.2 |
| | 6 | 8 | 1 | 0 | 0 | 1 | 0.1 |
| | 24 | 9 | 0 | 0 | 0 | 0 | 0 |
| | 48 | 9 | 0 | 0 | 0 | 0 | 0 |

CLAIMS:

1. An ointment base characterized by containing, as a solvent for the medicament, an imidazolidinone derivative represented by the formula

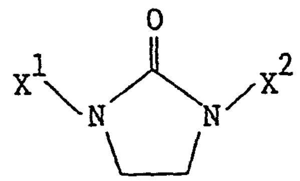

(I)

wherein $X^1$ and $X^2$ each represent a lower alkyl group.

2. An ointment base as defined in claim 1 which contains:

(a) a principal ointment component and

(b) an imidazolidinone derivative of the formula (I) or a mixture of an imidazolidinone derivative of the formula (I) and a cosolvent.

3. An ointment base as defined in claim 2 which contains:

(a) a principal ointment component;

(b) an imidazolidinone derivative of the formula (I) or a mixture of an imidazolidinone derivative of the formula (I) and a cosolvent; and when required

(c) a surfactant.

4. An ointment base as defined in claim 3 which contains:

(a) 30 to 99.9% by weight of petrolatum and/or polysiloxane;

(b) 0.1 to 30% by weight of an imidazolidinone derivative of the formula (I) or a mixture of an imidazolidinone derivative of the formula (I) and a cosolvent; and

(c) 45% by weight or less of a surfactant.

5. An ointment base as defined in claim 4 which contains:

(a) 60 to 99.9% by weight of petrolatum and/or polysiloxane;

(b) 0.5 to 30% by weight of an imidazolidinone derivative of the formula (I) or a mixture of an imidazolidinone derivative of the formula (I) and a cosolvent; and

(c) more than 0 but not more than 45% by weight of a surfactant.

6. An ointment base as defined in claim 5 which contains 0.1 to 10% by weight of a surfactant.

7. An ointment base as defined in any one of claims 4 to 6 which further contains a thickener in an amount of more than 0 but not more than 20% by weight.

8. An ointment comprising the ointment base as defined in claim 1 and a therapeutically effective amount of a medicament.

9. An ointment as defined in claim 8 which contains an ointment base and a medicament in an amount

of 0.01 to 5.0 parts by weight per 100 parts by weight of the ointment base, the ointment base comprising:

(a) 30 to 99.9% by weight of petrolatum and/or polysiloxane;

(b) 0.1 to 30% by weight of an imidazolidinone derivative of the formula (I) or a mixture of an imidazolidinone derivative of the formula (I) and a cosolvent; and

(c) 45% or less by weight of a surfactant.

10. An ointment as defined in claim 9 in which the ointment base comprises:

(a) 60 to 99.9% by weight of petrolatum and/or polysiloxane;

(b) 0.5 to 30% by weight of an imidazolidinone derivative of the formula (I) or a mixture of an imidazolidinone derivative of the formula (I) and a cosolvent; and

(c) more than 0 but not more than 45% by weight of a surfactant.

11. An ointment as defined in claim 9 in which the medicament is a steroid.

12. An ointment as defined in claim 11 in which the medicament is chloromethyl 17α-ethoxycarbonyloxy-9α-fluoro-11β-hydroxy-16α-methylandrosta-1,4-diene-3-one-17β-carboxylate.

13. An ointment as defined in claim 11 in which the medicament is chloromethyl 17α-ethoxycarbonyloxy-11β-hydroxyandrosta-1,4-diene-3-one-17β-carboxylate.

FIG. 1